# EUROPEAN PATENT SPECIFICATION

(11) **EP 3 989 964 B1**
(45) Date of publication and mention of the grant of the patent: **19.02.2025**
(21) Application number: 20742604.0
(22) Date of filing: 29.06.2020
(51) Int. Cl.: A61K 31/343, A61K 31/55, A61K 33/00, A61P 25/00, A61P 43/00

(54) **TASIMELTEON USE IN TREATING SLEEP ABERRATIONS**
TASIMELTEONVERWENDUNG ZUR BEHANDLUNG VON SCHLAFSTÖRUNGEN
UTILISATION DE TASIMELTÉON DANS LE TRAITEMENT D'ABERRATIONS DU SOMMEIL

(30) Priority: 29.06.2019 US 201962868881 P
(43) Date of publication of application: 04.05.2022
(73) Proprietor: Vanda Pharmaceuticals Inc., Washington, DC 20037 (US)
(72) Inventor: POLYMEROPOULOS, Mihael, Potomac, MD 20854 (US); SMIESZEK, Sandra, Washington, DC 20037 (US)
(74) Representative: Appleyard Lees IP LLP
(86) International application number: PCT/US2020/040081
(87) International publication number: WO 2021/003086

(56) References cited:
- BONACCI J M ET AL: "Tasimelteon (Hetlioz(TM)): A new melatonin receptor agonist for the treatment of non-24-hour sleep-wake disorder", JOURNAL OF PHARMACY PRACTICE, TECHNOMIC PUBLISHING CO, US, vol. 28, no. 5, 22 October 2015 (2015-10-22), pages 473 - 478, XP009520099, ISSN: 0897-1900, DOI: 10.1177/0897190014544792
- POLYMEROPOULOS C ET AL: "P328: Tasimelteon for jet lag disorder: results of the JET8 study, a randomized placebo controlled phase 3 trial", JOURNAL OF SLEEP RESEARCH; 24TH CONGRESS OF THE EUROPEAN SLEEP RESEARCH SOCIETY, ESRS 2018, ELSEVIER/WILEY, US; BASEL, CH, vol. 27, no. Supplement 1, September 2018 (2018-09-01), pages 240, XP009513265, ISSN: 1365-2869, [retrieved on 20180911], DOI: 10.1111/JSR.12751
- PATKE ALINA ET AL: "Mutation of the Human Circadian Clock GeneCRY1in Familial Delayed Sleep Phase Disorder", CELL, ELSEVIER, AMSTERDAM, NL, vol. 169, no. 2, 6 April 2017 (2017-04-06), pages 203, XP029970794, ISSN: 0092-8674, DOI: 10.1016/J.CELL.2017.03.027
- MARIA NOVELLA ET AL: "HCN Channels Modulators: The Need for Selectivity", CURRENT TOPICS IN MEDICINAL CHEMISTRY, 2016, pages 1764 - 1791, XP055666478, Retrieved from the Internet <URL:https://pdfs.semanticscholar.org/97e3/6620a92fd59dfcca8cdb2368cfc0a2382c0e.pdf?_ga=2.30615672.610542076.1601453016-2062215056.1594114700> [retrieved on 20200207], DOI: 10.2174/1568026616999160315130832
- SMIESZEK S ET AL: "Large whole genome sequencing study identifies novel variants associated with intrinsic circadian period in humans", SLEEP MEDICINE, ELSEVIER, AMSTERDAM, NL, vol. 64, December 2019 (2019-12-01), XP085952340, ISSN: 1389-9457, DOI: 10.1016/J.SLEEP.2019.11.993
- MARIA ANTONIA QUERA SALVA ET AL: "Non-24-Hour Sleep-Wake Rhythm Disorder in the Totally Blind: Diagnosis and Management", FRONTIERS IN NEUROLOGY, vol. 8, 18 December 2017 (2017-12-18), XP055734632, ISSN: 1664-2295, DOI: 10.3389/fneur.2017.00686

## Description

### CROSS-REFERENCE TO RELATED APPLICATIONS

This application claims the benefit of co-pending US Provisional Patent Application Serial No. 62/868,881, filed 29 June 2019.

### BACKGROUND OF THE INVENTION

The present invention provides new uses of tasimelteon in the treatment of sleep aberrations. In particular, the present invention relates to the use of tasimelteon in patients with certain identified genetic markers.

Circadian rhythms affect many aspects of human physiology, including a wide range of molecular and behavioral processes. Alterations in the timing or misalignment of circadian rhythms can result in various untoward effects.

The intrinsic period of the human circadian pacemaker averages greater than the 24-hour day, *i.e.,* averages 24.18 hours. While the circadian pacemaker operates in healthy individuals to synchronize the individual's day-night sleep cycle to a 24-hour day with a normal daily timing for onset of the individual's sleep period, other "chronotypes" can produce aberrant day-night sleep cycles. Both "morning" and "evening" chronotypes are known sleep aberrations. For individuals exhibiting these chronotypes, there is a propensity for a daily early or delayed sleep timing, respectively, relative to the sleep timing that would normally be experienced by an individual with a synchronized circadian pacemaker (i.e. an individual with a natural chronotype or natural sleep time).

The evening chronotype (delayed onset of sleep time) has been associated with greater morbidity, including higher rates of metabolic dysfunction and cardiovascular disease. This chronotype is also associated with a higher incidence of type II diabetes and obesity, as compared to the morning chronotype (advanced onset of sleep time). The evening chronotype has also been associated with an increased likelihood of depression. Similar associations have been found among those engaged in shift work, where sleep is delayed with respect to an individual's natural chronotype or natural sleep timing.

Sleep aberrations also include having a circadian period (tau) that is shortened, *i.e.,* less than 24 hours, such that sleep cycles are less than 24 hours apart.

Genetic variations underly a number of circadian rhythm and sleep disorders. Delayed Sleep Phase Disorder (DSPD), for example, has been associated with a CRY 1 splicing variant. See Patke et al., Mutation of the Human Circadian Clock Gene CRY1 in Familial Delayed Sleep Phase Disorder, Cell 169(2):203-215.e13 (2017). Several large genome-wide association studies (GWASs) have found correlations between a number of chronotype-associated loci and mental health disorders. See Jones et al. Genome-wide association analyses of chronotype in 697,828 individuals provides insights into circadian rhythms, Nature Communications 10:343 (2019) and references cited therein.

Sleep aberrations may also include drug-induced delayed sleep time, where an individual exhibits a delayed sleep time similar to that observed in DSPD as a result of the effects of one or more active agents.

Hyperpolarization-activated cyclic nucleotide-gated (HCN) channels are integral membrane proteins that mediate rhythmic electrical activity of cardiac pacemaker cells and, in neurons, play important roles in setting resting membrane potentials, dendritic integration, neuronal pacemaking, and the establishment of action potential thresholds. Because of their role in generating rhythmic activity in heart and brain cells, HCN channels are often referred to as pacemaker channels. See Bender et al., Hyperpolarization activated cyclic-nucleotide gated (HCN) channels in developing neuronal networks, Prog. Neurobiol. 86(3): 129-140 (2008).

In humans, four HCN channel proteins are known-HCN1, HCN2, HCN3, and HCN4. HCN1 is highly expressed in the brain and may modulate excitability in the brain. Specifically, the HCN1 protein is expressed in the frontal cortex and the retina and plays a critical role in shaping the autonomous activity of single neurons and the periodicity of network oscillations.

Tasimelteon (marketed under the trademark HETLIOZ^{®}), and pharmaceutical compositions and uses thereof, have been described in the art. See US Patent No. 5,856,529 and the compound specifically described in claim 7 therein. Tasimelteon is approved for use as a human medicine for the treatment of Non-24-Hour Sleep-Wake Disorder (Non-24) and is available in a 20 mg unit pharmaceutical dosage form (capsules), indicated for use prior to bedtime at the same time every night. Pharmacologically, tasimelteon is an agonist of the MT1R and MT2R melatonin receptors in the suprachiasmatic nucleus (SCN), the region of the brain associated with the biological clock. Engagement of these receptors by melatonin is believed to regulate circadian rhythms, including the sleep/wake cycle. Consistent with its receptor binding profile, tasimelteon demonstrates potent chronobiotic activity in preclinical models of acute phase-shifting and chronic re-entrainment.

US Patent No. 5,856,529 further claims a genus of compounds of which tasimelteon is a member for use in treating sleep disorders, as well as circadian rhythm disorders, in patients by administering an effective amount of tasimelteon. The patent describes tasimelteon as a melatonin agonist and states that melatonin agonists would be useful for the further study of melatonin receptor interactions as well as in the treatment of conditions affected by melatonin activity. The patent lists depression, jet lag, work-shift syndrome, and sleep disorders, among other possible therapeutic uses. Elsewhere the patent discloses that compounds within the genus of compounds of which tasimelteon is a member are useful as melatonergic agents in the treatment of sleep disorders, seasonal depression, shifts in circadian cycles, melancholia, stress, appetite regulation, benign prostatic hyperplasia and related conditions.

In addition to tasimelteon's approved dosing of 20 mg per day prior to bedtime at the same time every day, WO2007/137244 reports the discovery that effective human doses for tasimelteon can range from 10 to 100 mg/day for contemplated uses in sleep disorders and circadian rhythm disorders, with a further description that the exact dosing may be dependent upon particle size of the tasimelteon and the body size of the patient being treated. The publication also describes a 20 mg oral unit dosage form for tasimelteon and a clinical trial using tasimelteon in 10 mg, 20 mg, 50 mg, and 100 mg daily doses.

In US Patent Application Publication No. 20090105333A1 (WO2007/137244), results are reported from the aforementioned clinical trial in which tasimelteon was studied in subjects with a 5-hour advance in their sleep-wake cycle, *i.e.,* the type of sleep-wake cycle advance that might be experienced by a subject traveling by jet aircraft across the Atlantic Ocean from New York to London, including that treatment relative to placebo produced positive outcomes for shifting dim light melatonin onset and sleep efficacy.

Bonacci JM et al (Journal of Pharmacy Practice, 2015, 28(5): 473-478) discloses the treatment of non-24 sleep-wake disorder with tasimelteon.

Polymeropoulos C et al (Journal of Sleep Research, 2018, 27(Supplement 1): 240) discloses the treatment of jet lag disorders with tasimelteon.

### SUMMARY OF THE INVENTION

The present invention provides, in certain aspects, methods for treating individuals experiencing sleep aberrations. In particular, aspects of the present invention provide methods for use of tasimelteon for treating such individuals. The sleep aberrations addressed by the methods particularly relate to those individuals having certain single nucleotide polymorphisms (SNPs).

One aspect of the invention provides tasimelteon for use in the treatment of delayed sleep time in an individual, said treatment comprising: determining or having determined from a biological sample of the individual that the individual has a GG genotype at the rs12188518 single nucleotide polymorphism (SNP) locus, or the individual has a CC genotype at the rs11248864 single nucleotide polymorphism (SNP) locus, wherein tasimelteon is administered once daily before a target bedtime; and wherein the treatment is effective to advance the sleep time of the individual.

Another aspect of the invention provides tasimelteon for use in the treatment of a circadian rhythm disorder, said treatment comprising: administering to the individual a quantity of tasimelteon effective to shorten a circadian period (tau) in an individual, wherein the individual is selected from a group consisting of: individuals having an AA genotype at the rs72762058 single nucleotide polymorphism (SNP) locus.

The individuals being treated in accordance with the methods herein are human beings. In particular, the individuals being treated are those with a particular genotype at one or more of the rs12188518; rs72762058, or rs 11248864 SNP loci. These SNPs, identified by their "rs" SNP locus designations, are found on human chromosome 5, *i.e.,* chr5:46086964 (GRCh38.p12) (A>G; A>T) and chr5:45467324 (GRCh38.p12) (G>A), or chromosome 16 (*i.e.,* chr16: 1303514 (GRCh38.p12) (T>C; T>G). See, *e.g.,* https: //www.ncbi.nlm.nih.gov/snp/rs12188518, https://www.ncbi.nlm.nih.gov/snp/rs72762058, and https://www.ncbi.nlm.nih.gov/snp/rs11248864, respectively.

One skilled in the art will understand that the "rs" designators employed herein refer to "reference SNP ID" numbers, an identification system employed by NCBI to refer to a group or cluster of SNPs that map to an identical location. Other identification systems may be employed to refer to these chromosomal locations, as will be appreciated by one skilled in the art.

As used herein the term "sleep aberration" can include, for example, an abnormal pattern of sleep in an individual, which may take the form of a pattern of abnormally delayed onset of sleep or sleepiness (*i*.*e*., "sleep time"), an abnormally early onset of sleep time, an abnormally long or abnormally short daily sleep period (*i.e.,* substantially less than or substantially greater than a normal daily period of 7-9 hours in duration that an individual would set aside daily for sleep), or abnormal periods of prolonged daytime sleep or sleepiness as part of the individual's day-night sleep cycle.

One common form of sleep aberration results when an individual has a pattern of delayed sleep time relative to the sleep time that would normally be appropriate for a day-night sleep cycle for the individual. As noted above, "sleep aberration" may also include a shortened (*i*.*e*. less than 24-hour) tau or drug-induced delayed sleep time.

The determination of an individual's SNP status at a particular SNP locus is undertaken by methods known in the art for determining single nucleotide polymorphisms. In this regard, standard genetic testing is done using known techniques for analyzing a biological sample from an individual.

The treatment regimen described herein of administering tasimelteon is undertaken by administration before bedtime, using treatment regimens known in the art for the treatment of individuals with non-24. In this regard, the administration can be undertaken up to about 2 hours before the individual's target bedtime. Most typically the administration is undertaken about 0.5 to 1.5 hours before the target bedtime, e.g., about 1 hour before the target bedtime.

The administration of tasimelteon orally is preferred. Typically, a solid oral dosage form is used, e.g., a single capsule containing the daily dose of tasimelteon to be administered, although other routes of administration and dosage forms providing an equivalent effect can be used.

The amount of tasimelteon can vary based upon an individual's clinical response, although daily doses of 10 mg to 100 mg are typically effective under the treatment regimens herein. The preferred doses for treatment of adults are typically 20 mg to 50 mg daily, with 20 mg daily representing a typical effective dose.

When the tasimelteon is administered using an oral dosage form, any of the typical methods for administration can be used. Suitable dosage forms include liquid oral dosage forms and solid oral dosage forms, such as conventional compressed tablets and capsules.

### DETAILED DESCRIPTION

The present invention can be further understood through the following examples.

### Example 1:

### Chronotype Prediction

A first whole genome sequencing study is conducted using 316 samples collected as part of a clinical study directed to the effects of abrupt circadian advance, as may be experienced during eastward jet travel, and the ability of tasimelteon to mitigate or eliminate those effects. Participants in that study are healthy, sighted individuals not known to be suffering from a circadian rhythm disorder or sleep disorder.

More than 400 single nucleotide polymorphisms (SNPs) in linkage disequilibrium are found. Among the SNPs that are identified in this study, rs121888518, located within the HCN1 gene on chromosome 5, is found to be predictive of evening chronotype when compared to individuals' Morningness-Eveningness Questionnaire (MEQ) scores. Specifically, individuals with a GG genotype at the rs121888518 SNP locus are significantly more likely to be classified as having an evening chronotype based on MEQ score. This association is persistent in a binary analysis (logistic regression) and when using alternative morningness-eveningness questionnaires. Expression quantitative trait locus (eQTL) analysis indicates a significant result (1.6•10⁻⁹) for the rs 121888518 SNP

These results are consistent with a mechanism of action by which individuals exhibit an evening chronotype.

The HCN1 channel is responsible for feedback on the rods, regulating the dynamic range of light reactivity under dim or intermediate light conditions. Proper cone vision under mesopic conditions requires rapid adaptational feedback modulation of rod output via the HCN1 channels. When these channels are absent or inhibited, sustained rod responses following bright light exposure saturate the retinal network, resulting in a loss of downstream cone signaling.

The improper functioning of this feedback system in an individual may result in rod saturation, even in dim light. This may result in a misperception of light conditions and a consequent circadian delay, experienced as a sleep aberration, which may include delayed sleep time.

Thus, resetting the circadian period of an individual with a genetic predisposition to having an evening chronotype (e.g., GG genotype at the rs121888518 SNP locus) provides an avenue to eliminate or ameliorate the consequences of such a sleep aberration. Administering to such an individual a dose of tasimelteon before a target bedtime operates to advance the sleep time in the individual, thereby treating the sleep aberration.

Another SNP, rs 11248864, located on chromosome 16, similarly exhibits a significant correlation (p < 10⁻⁸) with chronotype. Specifically, individuals having a CC genotype at the rs 11248864 SNP locus are significantly more likely to be classified as having an evening chronotype based on MEQ score (eQTL=1.3•10⁻⁸).

The rs11248864 SNP is located within the Ubiquitin Conjugating Enzyme E2 I (UBE2I) gene. The UBE2I enzyme directly interacts with transcriptional repressor BHLHE40 and is potentially of direct relevance to the input and output of the circadian clock. The UBE2I region is also a significant eQTL for brain-specific angiogenesis inhibitor (BAIAP3), which in turn likely plays a role in hypothalamic neuronal firing by modulating gamma-aminobutyric acid (GABA)ergic inhibitory neurotransmission and is highly expressed in the pituitary.

As noted above, resetting the circadian period of an individual with a genetic predisposition to having an evening chronotype provides a method to eliminate or ameliorate the consequences of such a sleep aberration. Thus, administering to such an individual (*e.g*., one having a CC genotype at the rs11248864 SNP locus) a dose of tasimelteon before a target bedtime operates to advance the sleep time in the individual, thereby treating the sleep aberration.

This mechanism of action also suggests a method by which to delay sleep time in an individual. For example, an individual not predicted to have an evening chronotype (*e.g.,* an individual having an AA or AG genotype at the rs12188518 SNP locus or a TT or TC genotype at the rs 11248864 SNP locus) may be administered an HCN channel inhibitor in an amount effective to induce in such an individual a saturation of the rods under dim or intermediate light conditions, resulting in a delayed sleep time. Suitable HCN inhibitors include, for example, ivabradine, cilobradine, zatebradine, or cesium.

### Example 2:

### HCN1 and Prolonged Tau

A second whole genome sequencing study is conducted of 174 totally blind individuals with Non-24-Hour Sleep-Wake Disorder (Non-24). Non-24 is a circadian rhythm disorder in which the master body clock runs either slightly shorter or, more commonly, slightly longer than 24 hours.

Within this group, an association is found between HCN1 variants and circadian period length (tau) as calculated from the measurement of urinary 6-sulphatoxymelatonin (aMT6s) rhythms, aMT6s being the major metabolite of melatonin. Specifically, the minor allele of the SNP rs72762058, representing a G-to-A mutation at position 45467426, shows a significant association with longer tau. Individuals with this mutation have a mean tau of 24.71 hours, 12 minutes longer than those not having the minor allele.

Thus, shortening the tau of an individual shown to have a genetic predisposition to having a long tau provides a method for treating circadian rhythm and sleep aberrations associated with a long tau. Administering to such an individual an effective amount of tasimelteon operates to shorten the individual's tau, resulting in elimination or amelioration of the circadian rhythm or sleep aberration.

## Claims

1. Tasimelteon for use in the treatment of delayed sleep time in an individual, said treatment comprising:
determining or having determined from a biological sample of the individual that the individual has a GG genotype at the rs12188518 single nucleotide polymorphism (SNP) locus, or the individual has a CC genotype at the rs11248864 single nucleotide polymorphism (SNP) locus,
wherein tasimelteon is administered once daily before a target bedtime; and wherein the treatment is effective to advance the sleep time of the individual.

2. Tasimelteon for the use of claim 1, wherein the tasimelteon is administered orally.

3. Tasimelteon for the use of claim 2, wherein the tasimelteon is administered 0.5 hour to 2 hours before the individual's target bedtime.

4. Tasimelteon for the use of claim 1, wherein the dose of tasimelteon is between about 10 mg and about 100 mg.

5. Tasimelteon for the use of claim 4, wherein the dose of tasimelteon is between about 20 mg and about 50 mg.

6. Tasimelteon for the use of claim 5, wherein the dose of tasimelteon is 20 mg.

7. Tasimelteon for the use of claim 1, wherein the individual has a GG genotype at the rs12188518 SNP locus.

8. Tasimelteon for the use of claim 1, wherein the individual has a CC genotype at the rs1128864 SNP locus.

9. Tasimelteon for use in the treatment of a circadian rhythm disorder, said treatment comprising:
administering to the individual a quantity of tasimelteon effective to shorten a circadian period (tau) in an individual, wherein the individual is selected from a group consisting of: individuals having an AA genotype at the rs72762058 single nucleotide polymorphism (SNP) locus.

10. Tasimelteon for the use of claim 9, wherein tasimelteon is administered orally.

11. Tasimelteon for the use of claim 10, wherein the tasimelteon is administered 0.5 hour to 2 hours before the individual's target bedtime.

12. Tasimelteon for the use of claim 9, wherein the dose of tasimelteon is between about 10 mg and about 100 mg.

13. Tasimelteon for the use of claim 12, wherein the dose of tasimelteon is between about 20 mg and about 50 mg.

14. Tasimelteon for the use of claim 13, wherein the dose of tasimelteon is 20 mg.

## Patentansprüche

1. Tasimelteon zur Verwendung bei der Behandlung von verzögerter Schlafzeit bei einem Individuum, wobei die Behandlung umfasst:
Bestimmen oder aus einer biologischen Probe des Individuums bestimmen lassen, ob das Individuum einen GG-Genotyp an dem rs12188518 Einzelnukleotid-Polymorphismus-Locus (SNP-Locus) aufweist oder ob das Individuum einen CC-Genotyp an dem rs11248864 Einzelnukleotid-Polymorphismus-Locus (SNP-Locus) aufweist,
wobei Tasimelteon einmal täglich vor einer angestrebten Schlafenszeit verabreicht wird; und wobei die Behandlung wirksam ist, um die Schlafenszeit des Individuums zu verlängern.

2. Tasimelteon zur Verwendung nach Anspruch 1, wobei das Tasimelteon oral verabreicht wird.

3. Tasimelteon zur Verwendung nach Anspruch 2, wobei das Tasimelteon 0,5 Stunden bis 2 Stunden vor der angestrebten Schlafenszeit des Individuums verabreicht wird.

4. Tasimelteon zur Verwendung nach Anspruch 1, wobei die Dosis von Tasimelteon zwischen etwa 10 mg und etwa 100 mg beträgt.

5. Tasimelteon zur Verwendung nach Anspruch 4, wobei die Dosis von Tasimelteon zwischen etwa 20 mg und etwa 50 mg beträgt.

6. Tasimelteon zur Verwendung nach Anspruch 5, wobei die Dosis von Tasimelteon 20 mg beträgt.

7. Tasimelteon zur Verwendung nach Anspruch 1, wobei das Individuum einen GG-Genotyp am SNP-Locus rs12188518 aufweist.

8. Tasimelteon zur Verwendung nach Anspruch 1, wobei das Individuum einen CC-Genotyp am SNP-Locus rs1128864 aufweist.

9. Tasimelteon zur Verwendung bei der Behandlung einer Störung des zirkadianen Rhythmus, wobei die Behandlung Folgendes umfasst:
Verabreichen einer Menge von Tasimelteon an das Individuum, die zur Verkürzung einer zirkadianen Periode (Tau) in einem Individuum wirksam ist, wobei das Individuum ausgewählt ist aus einer Gruppe, bestehend aus: Individuen, die einen AA-Genotyp am rs72762058-Einzel-Nukleotid-Polymorphismus-Locus (SNP-Locus) aufweisen.

10. Tasimelteon zur Verwendung nach Anspruch 9, wobei Tasimelteon oral verabreicht wird.

11. Tasimelteon zur Verwendung nach Anspruch 10, wobei das Tasimelteon 0,5 Stunden bis 2 Stunden vor der angestrebten Schlafenszeit des Individuums verabreicht wird.

12. Tasimelteon zur Verwendung nach Anspruch 9, wobei die Dosis von Tasimelteon zwischen etwa 10 mg und etwa 100 mg beträgt.

13. Tasimelteon zur Verwendung nach Anspruch 12, wobei die Dosis von Tasimelteon zwischen etwa 20 mg und etwa 50 mg beträgt.

14. Tasimelteon zur Verwendung nach Anspruch 13, wobei die Dosis von Tasimelteon 20 mg beträgt.

## Revendications

1. Tasimeltéon pour une utilisation dans le traitement du temps de sommeil retardé chez un individu, ledit traitement comprenant :
déterminer ou avoir déterminé à partir d'un échantillon biologique de l'individu que l'individu a un génotype GG au niveau du locus de polymorphisme mononucléotidique (SNP) rs12188518, ou que l'individu a un génotype CC au niveau du locus de polymorphisme mononucléotidique (SNP) rs11248864,
dans lequel le tasimeltéon est administré une fois par jour avant une heure cible de coucher ; et dans lequel le traitement est efficace pour faire avancer le temps de sommeil de l'individu.

2. Tasimeltéon pour l'utilisation selon la revendication 1, dans lequel le tasimeltéon est administré par voie orale.

3. Tasimeltéon pour l'utilisation selon la revendication 2, dans lequel le tasimeltéon est administré 0,5 heure à 2 heures avant l'heure cible du coucher de l'individu.

4. Tasimeltéon pour l'utilisation selon la revendication 1, dans lequel la dose de tasimeltéon est comprise entre environ 10 mg et environ 100 mg.

5. Tasimeltéon pour l'utilisation selon la revendication 4, dans lequel la dose de tasimeltéon est comprise entre environ 20 mg et environ 50 mg.

6. Tasimeltéon pour l'utilisation selon la revendication 5, dans lequel la dose de tasimeltéon est de 20 mg.

7. Tasimeltéon pour l'utilisation selon la revendication 1, dans lequel l'individu a un génotype GG au niveau du locus SNP rs12188518.

8. Tasimeltéon pour l'utilisation selon la revendication 1, dans lequel l'individu a un génotype CC au niveau du locus SNP rs1128864.

9. Tasimeltéon pour une utilisation dans le traitement d'un trouble du rythme circadien, ledit traitement comprenant :
l'administration à l'individu d'une quantité de tasimeltéon efficace pour raccourcir une période circadienne (tau) chez un individu, l'individu étant choisi dans un groupe constitué : des individus ayant un génotype AA au niveau du locus de polymorphisme mononucléotidique (SNP) rs72762058.

10. Tasimeltéon pour l'utilisation selon la revendication 9, dans lequel le tasimeltéon est administré par voie orale.

11. Tasimeltéon pour l'utilisation selon la revendication 10, dans lequel le tasimeltéon est administré 0,5 heure à 2 heures avant l'heure cible du coucher de l'individu.

12. Tasimeltéon pour l'utilisation selon la revendication 9, dans lequel la dose de tasimeltéon est comprise entre environ 10 mg et environ 100 mg.

13. Tasimeltéon pour l'utilisation selon la revendication 12, dans lequel la dose de tasimeltéon est comprise entre environ 20 mg et environ 50 mg.

14. Tasimeltéon pour l'utilisation selon la revendication 13, dans lequel la dose de tasimeltéon est de 20 mg.
